# EUROPEAN PATENT SPECIFICATION

(11) **EP 0 277 386 B1**
(45) Date of publication and mention of the grant of the patent: **14.06.1995**
(21) Application number: 87202613.3
(22) Date of filing: 23.12.1987
(51) Int. Cl.: C07C 205/04, C07C 201/16

(54) **A method for the production of HNS II**
Verfahren zur Herstellung von HNS II
Procédé pour la production de HNS II

(30) Priority: 21.01.1987 SE 8700212
(43) Date of publication of application: 10.08.1988
(73) Proprietor: Bofors Explosives AB, S-691 86 Karlskoga (SE)
(72) Inventor: Bellamy, Anthony, S-691 44 Karlskoga (SE)
(74) Representative: Falk, Bengt

(56) References cited:
- US-A- 3 505 413
- US-A- 3 510 266
- US-A- 3 699 176
- US-A- 4 604 489
- Carey, F.A. & Sundberg, R.J. "Advanced Organic Chemistry", 2nd Edition (1984), p. 268.
- Römpp Chemie Lexikon, 9th Edition (1989), p. 232.

## Description

This invention relates to a method for the production of HNS II, which is 2,2',4,4',6,6'-hexanitrostilbene (HNS) with specified purity, properties and bulk density, from a less pure raw-product, with lower bulk density, usually referred to as HNS I.

The search for explosives which retain their performance characteristics after being subjected to high temperatures for long periods, for use in for example space technology and natural oil/gas recovery, has focused to a large extent upon 2,2',4,4',6,6'-hexanitrostilbene (HNS) as a promising candidate. The purified explosive has a melting point of 319°C and exhibits virtually unchanged explosive properties after being heated at 232°C for 200h. Furthermore it is easily manufactured from the readily available 2,4,6-trinitrotoluene (TNT) according to the process described in US 3 505 413. The product so obtained, HNS I, is not however pure, containing small amounts of TNT, hexanitrobibenzyl, and other impurities, and consequently its properties are somewhat less than optimal. Its melting point is usually around 315°C and its thermal stability is significantly reduced from that of the purified explosive HNS II. The product HNS II is defined by its purity and its bulk density. The present invention concerns only a method for the production of a HNS with the purity of HNS II from the less pure raw product of HNS I.

During the past 20 years considerable effort has been devoted to finding methods for purifying HNS I to the high quality HNS II, which has the specifications listed in Table 1. While several methods purifying HNS I have now been published, many of them give HNS below the specification limits for HNS II in one or more respects. All of these methods involve either recrystallisation or digestion of the HNS I using either an organic solvent system or nitric acid, sometimes followed by physical processing of the crystals.

Since HNS has a rather low solubility in most organic solvents, the choice of solvents for recrystallisation is rather limited. Most work has focused on the use of dimethylformamide (DMF), 90% HNO₃, and to some extent acetonitrile. The use of DMF, alone or in conjunction with acetonitrile, gives HNS in the form of long needles with a rather low bulk density (0.25-0.45gcm⁻³). However the handling characteristics can be improved by milling, the crystals being fractured and the bulk density increased to around 0.55gcm⁻³. Unfortunately the main limitation of this method is the use of DMF as the solvent ; whilst most of the specification requirements for HNS II are satisfied, the use of DMF almost invariably leads to vacuum test results which exceed the specification limit ( <0.6mlg⁻¹ for 20min at 260°C; the value at 2h is usually within the specification limit). The use of digestion rather than recrystallisation is also only partially successful. Whilst most of the impurities are removed by this method, the bulk density cannot reliably be increased above 0.45gcm⁻³. Recrystallisation from 90% HNO₃ is widely used in the manufacture of HNS II because it is relatively cheap and easy to perform. However it is generally felt that the resulting product is almost always contaminated with nitric acid. E.E. Kilmer (NSWC/WOL TR 78-209; also 75-142) has made a thorough study of this question and demonstrated that HNO₃ can be effectively removed from the crystals by proper washing and drying (120°C in vacuo - not in air). He concludes however that fulfillment of the vacuum test requirements is much easier when organic solvents such as acetonitrile/toluene or acetonitrile/xylene are used. Kilmer also studied the performance of detonating cord filled with HNS II which had been recrystallised from either 90% HNO₃ or acetonitrile/toluene or acetonitrile/xylene, after thermal treatment at 218°C (425°F), or repeated cycling between -54°C (-65°F) and 177°C (350°F). With HNS II which contained as little as 0.01% residual HNO₃, he observed decreased detonation velocity after repeated thermal cycling, or even after normal storage for 4 years, and decreased detonation velocity (failure after 20h) and chemical degradation (<20% HNS remained after 20h) on heating at 218°C. By contrast, HNS II obtained by acetonitrile/toluene or acetonitrile/xylene recrystallisation, was resistent to themal treatment viz. no decrease in the detonation velocity was observed after 264h at 218°C, or after 100 thermal cycles.

The most satisfactory, reported method for producing HNS II with regard to product specification is that described by L.J. Syrop in US 3 699 176 and US 3 832 142. This consists of a continuous extraction process, in which hot acetonitrile extracts HNS from solid HNS I and this solution then passes into a higher boiling , non-solvent for HNS eg.toluene or xylene. The HNS thus separates from the boiling aoetonitrile/toluene or acetonitrile/xylene mixture as HNS II, and the acetonitrile is returned to the extraction cycle. This method gives excellent HNS II with bulk density around 0.5gcm⁻³, melting point 319°C, and good vacuum stability (260°C), but is rather slow due to the low solubility of HNS in acetonitrile.

The present invention avoids the deficiencies of the above reported methods for producing HNS II by using N-methylpyrrolidone (1-methyl-2-pyrrolidone) as the recrystallising solvent for the production of a HNS with the purity of HNS II.

**Table 1**

| | HNS purified according to the invention | Starting HNS (HNS I) | Specification for HNS II** |
|---|---|---|---|
| Chemical analysis: | | | |
| Hexanitrobibenzyl (%) | <0.01 | 0.22 | <1.2 |
| 2,4,6-Trinitrotoluene (%) | <0.01 | 0.01 | |
| Others (%) | undetectable | 0.49 | 0.05 |
| Surface moisture (wt%) | <0.01 | - | <0.05 |
| Solubility in water (wt%) | 0.02 | 0.1-0.2 | <0.03 |
| Insoluble in DMF (wt%) | <0.01 | 0.09 | <0.03 |
| Acidity (Δ pH from control) | <-0.2 | >-0.2 | <-0.2 |

| Vacuum stability (260°C) | | | |
|---|---|---|---|
| 1st 20min (mlg⁻¹) | 0.54 | 4.6 | <0.6 |
| additional 2h (mlg⁻¹h⁻¹) | 0.41 | 2.4 | <0.6 |

| | | | |
|---|---|---|---|
| * according to DIN 53 194 | | | |
| ** according to WS 5003F | | | |

N-Methylpyrrolidone has previously been used by E.E. Gilbert as a solvent for the oxidation of hexanitrobibenzyl to HNS (Propellants and Explosives, 1980, 5, 168; US 4 245 129; US 4 243 614; US 4 270 012; US 4 268 696), and for the conversion of TNT to hexanitrobibenzyl, replacing THF in the traditional Shipp-Kaplan process (Propellants and Explosives, 1980, 5, 15), but its use as a recrystallising solvent for HNS has not been reported. Although quite structurally similar to DMF, N-methylpyrrolidone has almost twice the DMF-solvent capacity for HNS (solubility gHNS/100ml N-methylpyrrolidone : 4.3g at 10°C, 11.1g at 100°C, 17.8g at 125°C cf 6.1g HNS/100ml DMF at 100°C), and gives a product which has good vacuum stability at 260°C (see Table 1). The latter is the main limitation of DMF. The solubility data indicate that N-methylpyrrolidone should give recovery yields of 61% and 76% when saturated solutions of HNS are cooled from 100°C and 125°C resp. to 10°C. Example 1 indicates that recoveries close to the theoretical are readily attainable. Recovery yields can be increased further by the addition of a solubility reducing cosolvent such as chlorobenzene (Examples 2 and 3 ; 85.5 and 85% resp.) or toluene (Example 4; 82%), without raising the vacuum stability at 260°C above the specification limit.

The HNS obtained by recrystallistaion from N-methylpyrrolidone, with or without a cosolvent, is in the form of needles (see Fig 1) whose length to breadth ratio is dependent upon the temperature profile of the cooling cycle, and the rate of addition of the solubility reducing cosolvent when used. Typical bulk densities are 0.3-0.45gcm⁻³, with volume mean diameters (VMD) of 100-250»m. As such, the bulk density is below the specification limit for HNS II (< 0.450gcm⁻³), and the material has poor flow and handling characteristics. The bulk density can be increased, accompanied by a decrease in VMD, and the flow and handling characteristics greatly improved, by ultrasonic treatment of a suspension (slurry) of the recrystallised HNS.

A complete characterisation of the product obtained by recrystallisation of HNS from N-methylpyrrolidone + toluene at 125°C, is shown in Table 1, together with the corresponding analyses of the starting HNS I and the specification for HNS II. It should be noted that the final product satisfies the specification for purity for HNS II.

### Example 1

HNS (200g) was recrystallised by dissolution in N-methylpyrrolidone (1125ml) at 125°C and then slow cooling to 10°C. The solid was filtered off, was washed with MeOH (2X) and 3%MeOH in H₂O (3X), and then dried. Yield: 147g (73.5%). Bulk density 0.42gcm⁻³, VMD 251»m. Vacuum test (260°C): 0.44mlg⁻¹ after 20min, 0.27mlg⁻¹h⁻¹ after 2h.

### Example 2

HNS (400g) was dissolved in N-methylpyrrolidone (2250ml) at 125°C. PhCl (2250ml) was then added with mechanical stirring during 50min while keeping the temperature at 125°C. The mixture was cooled in air to 65°C and then in ice/water to 10°C. The solid was filtered off, was washed with MeOH (2X) and 3% MeOH in H₂O (3X), and then dried. Yield:342g (85.5%). Bulk density 0.48gcm⁻³, VMD 184»m. Vacuum test (260°C): 0.38mlg⁻¹ after 20min, 0.29 mlg⁻¹h⁻¹ after 2h.

### Example 3

HNS (400g) was recrystallised from N-methylpyrrolidone and PhCl as in Example 2, except that cooling from 125°C was with ice/water throughout. Yield: 340g (85%). Bulk density 0.44gcm⁻³, VMD 193»m. Vacuum test (260°C): 0.51mlg⁻¹ after 20min, 0.12mlg⁻¹h⁻¹ after 2h.

### Example 4

HNS (400g) was recrystallised as in Example 3, except that PhCH₃ was used instead of PhCl. Yield: 328g (82%). Bulk density 0.30gcm⁻³, VMD 135»m. Vacuum test (260°C): 0.51mlg⁻¹ after 20min, 0.10mlg⁻¹h⁻¹ after 2h.

## Claims

1. A method for the production of a 2,2',4,4',6,6'-hexanitrostilbene (HNS) with the specified purity of HNS II from a less pure raw-product, generally referred to as HNS I, comprising recrystallisation of the above rawproduct from N-methylpyrrolidone (1-methyl-2-pyrrolidone).

## Patentansprüche

1. Verfahren zur Herstellung von 2,2',4,4',6,6'-Hexanitrostilben (HNS) mit der spezifizierten Reinheit von HNS II aus einem weniger reinen Rohprodukt, allgemein als HNS I bezeichnet, umfassend eine Rekristallisation des Rohproduktes aus N-Methylpyrrolidon (1-Methyl-2-pyrrolidon).

## Revendications

1. Procédé de production de 2,2',4,4',6,6'-hexanitrostilbène (HNS) ayant la pureté spécifiée pour HNS II, à partir d'un produit brut moins pur, appelé de façon générale HNS I, ce procédé comprenant (ou consistant en) une recristallisation du produit brut ci-dessus à partir de N-méthylpyrrolidone (1-méthyl-2-pyrrolidone).
